(19) Europäisches Patentamt
European Patent Office
Office européen des brevets

(11) EP 4 230 191 A1

(12) **EUROPEAN PATENT APPLICATION**
published in accordance with Art. 153(4) EPC

(43) Date of publication:
**23.08.2023 Bulletin 2023/34**

(21) Application number: **21880052.2**

(22) Date of filing: **11.10.2021**

(51) International Patent Classification (IPC):
***A61K 8/37*** (2006.01) ***A61K 8/06*** (2006.01)
***A61K 8/19*** (2006.01) ***A61K 8/31*** (2006.01)
***A61K 8/49*** (2006.01) ***A61K 8/891*** (2006.01)
***A61K 8/894*** (2006.01) ***A61Q 1/02*** (2006.01)

(52) Cooperative Patent Classification (CPC):
**A61K 8/06; A61K 8/19; A61K 8/31; A61K 8/37; A61K 8/49; A61K 8/58; A61K 8/81; A61K 8/891; A61K 8/894; A61Q 1/00; A61Q 1/02**

(86) International application number:
**PCT/JP2021/037568**

(87) International publication number:
**WO 2022/080313 (21.04.2022 Gazette 2022/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.10.2020 JP 2020174263**

(71) Applicant: **Kao Corporation**
**Chuo-ku**
**Tokyo 103-8210 (JP)**

(72) Inventors:
- **TATEBE, Momoko**
  **Odawara-shi, Kanagawa 250-0002 (JP)**
- **FUJIOKA, Masahiro**
  **Odawara-shi, Kanagawa 250-0002 (JP)**

(74) Representative: **Hoffmann Eitle**
**Patent- und Rechtsanwälte PartmbB**
**Arabellastraße 30**
**81925 München (DE)**

(54) **EMULSION COMPOSITION**

(57) An emulsion composition comprising the following components (A), (B), (C), (D), and (E): (A) a lipophilic nonionic surfactant; (B) a film-forming agent; (C) a nonvolatile oil which is liquid at 25°C; (D) a carbonate; and (E) water.

EP 4 230 191 A1

## Description

Field of the Invention

The present invention relates to an emulsion composition.

Background of the Invention

For makeup cosmetics such as foundations, it is necessary to firmly adhere the cosmetic to the skin in order to cover the pores.

For example, Patent Literature 1 states that a water-in-oil emulsion cosmetic containing a composite powder containing particles in which the surfaces of silicone rubber particles are coated with a silicone resin, and a nonvolatile oil agent other than a silicone oil makes the skin bright, is excellent in extensibility and close adhesiveness, and exhibits a pore hiding effect.

Meanwhile, sunscreen cosmetics containing carbonates are known.

For example, Patent Literature 2 describes a sunscreen cosmetic containing an inorganic ultraviolet scattering agent containing a hydrophobic fine particle metal oxide, tubular basic magnesium carbonate, and an oil, and Patent Literature 3 describes a cosmetic composition having UV-A and/or UV-B protection containing at least one inorganic UV filter and surface-reacted calcium carbonate.

In these cosmetics, carbonates are used to improve the ultraviolet protection effect.

(Patent Literature 1) JP-A-2014-172872
(Patent Literature 2) JP-A-2011-236182
(Patent Literature 3) JP-A-2020-506953

Summary of the Invention

The present invention relates to an emulsion composition comprising the following components (A), (B), (C), (D), and (E):

(A) a lipophilic nonionic surfactant;
(B) a film-forming agent;
(C) a nonvolatile oil which is liquid at 25°C;
(D) a carbonate; and
(E) water.

Detailed Description of the Invention

Conventional emulsion cosmetics tend to give a sliding feeling on the skin during application, an actual feeling that the cosmetic is sufficiently adhered to the skin is insufficient, and the adhesion of the cosmetic to the pores is also weak, and the pores cannot be sufficiently covered. Another problem is that a sticky feeling remains on the skin after application.

The present inventors found that an emulsion composition which solves the above problems is obtained by using a carbonate in combination with a lipophilic nonionic surfactant, a film-forming agent, and a nonvolatile oil which is liquid at 25°C.

With the emulsion composition of the present invention, the sliding feeling is reduced during application, the feeling of close adhesion to the skin after application is improved, and the pores can be sufficiently covered, and moreover the sticky feeling on the skin after application is reduced.

The sliding feeling indicates that when an emulsion cosmetic is taken on a fingertip or the like and applied to the face, there is a feeling that the emulsion cosmetic spreads for a long period of time on the skin as if a liquid oil agent were spread, and there is no actual feeling that the emulsion cosmetic spreads while adhering to the skin. The feeling of close adhesion indicates that an emulsion composition attached to the skin after application follows the movement of the skin but does not come off the surface of the skin even if the skin of the face is moved by a change in expression, or the like; and the emulsion composition does not gather partially in the form of large wrinkles, small wrinkles, and the like; and the emulsion composition adheres firmly.

The component (A) lipophilic nonionic surfactant used in the present invention preferably has an HLB of from 1 to 8, more preferably from 2 to 7, and further more preferably from 3 to 6 from the viewpoint of reducing the sliding feeling of the emulsion composition on the skin during application, improving the feeling of close adhesion after application,

and obtaining a stable emulsion composition.

Here, HLB (Hydrophilic-Lipophilic Balance) represents the molecular weight of the hydrophilic group moiety in the total molecular weight of the surfactant and is obtained by Griffin's equation. When the component (A) is composed of two or more nonionic surfactants, the HLB of the surfactant mixture is obtained as follows. The HLB of the surfactant mixture is the arithmetic mean of the HLB values of the nonionic surfactants based on their blend ratio.

$$\text{HLB for mixture} = \Sigma(\text{HLBx} \times \text{Wx})/\Sigma\text{Wx}$$

HLBx represents the HLB value of a nonionic surfactant X.
Wx represents the mass (g) of the nonionic surfactant X having the value of HLBx.

Examples of the component (A) lipophilic nonionic surfactant include glycerin fatty acid esters, propylene glycol fatty acid esters, sorbitan fatty acid esters such as sorbitan monoisostearate, sorbitan monooleate, sorbitan sesquistearate, and sorbitan sesquioleate, polyoxyethylene castor oil, polyoxyethylene hydrogenated castor oil, polyoxyethylene hydrogenated castor oil fatty acid esters, polyethylene glycol fatty acid esters, alkyl glyceryl ethers, alkyl polyglyceryl ethers, polyoxyethylene alkyl ethers, polyoxyethylene alkyl ether fatty acid esters, polyoxyethylene alkylamines, and silicone-based surfactants such as polyether-modified silicones, alkyl-modified polyether-modified silicones, and alkyl chain-co-modified polyether-modified silicones, such as polyoxyethylene-methylpolysiloxane copolymers and poly(oxyethylene-oxypropylene) methylpolysiloxane copolymers.

Among these, at least one or more selected from the group consisting of sorbitan fatty acid esters and silicone-based surfactants are preferably included, at least one or more selected from the group consisting of sorbitan fatty acid esters and polyether-modified silicones are more preferably included, at least one or more selected from the group consisting of sorbitan monoisostearate, sorbitan monooleate, sorbitan sesquistearate, and polyether-modified silicones are further more preferably included, and at least one or more selected from the group consisting of sorbitan monoisostearate and polyoxyethylene-methylpolysiloxane copolymers are even more preferably included, from the viewpoint of reducing the sliding feeling of a water-in-oil emulsion composition on the skin during application, improving the feeling of close adhesion to the skin after application, and obtaining a stable emulsion composition.

A sorbitan fatty acid ester and a silicone-based surfactant are preferably used in combination.

As the component (A) lipophilic nonionic surfactant, a commercial product can be used. Examples of sorbitan monoisostearate include Span 120 (HLB 4.7) (manufactured by Croda). Examples of the polyoxyethylene-methylpolysiloxane copolymers include KF-6015 (HLB 4.5), KF-6017 (HLB 4.5), and KF-6028 (HLB 4) (all manufactured by Shin-Etsu Chemical Co., Ltd.), and SH3775M (HLB 5) (manufactured by Dow Corning Toray Co., Ltd.). Examples of the poly(oxyethylene-oxypropylene) methylpolysiloxane copolymers include KF-6012 (HLB 7) (manufactured by Shin-Etsu Chemical Co., Ltd.), and DOWSIL BY 22-008 M (HLB 2) and ES-5226 DM formulation Aid (HLB 2) (all manufactured by Dow Corning Toray Co., Ltd.). Examples of the alkyl-modified polyether-modified silicones include KF-6038 (HLB 3) (manufactured by Shin-Etsu Chemical Co., Ltd.) and ABIL EM 90 (HLB 5) (manufactured by Evonik).

One or more lipophilic nonionic surfactants, components (A), can be used, and the content is preferably 1% by mass or more, more preferably 1.5% by mass or more, and further more preferably 2% by mass or more, and preferably 8% by mass or less, more preferably 6.5% by mass or less, and further more preferably 5.5% by mass or less in the total composition from the viewpoint of reducing the sliding feeling of the emulsion composition on the skin during application, improving the feeling of close adhesion to the skin after application, and obtaining a stable emulsion composition. The content of the component (A) is preferably from 1 to 8% by mass, more preferably from 1.5 to 6.5% by mass, and further more preferably from 2 to 5.5% by mass in the total composition.

The component (B) film-forming agent is one used in ordinary cosmetics, and examples of the film-forming agent include fluorine-modified silicone resins, trimethylsiloxysilicate, acrylic silicone resins, and alkylsilsesquioxane resins.

As the fluorine-modified silicone resin, preferred is one having a structure of the following formula (1):

$$R^1_gSiO_{(4-g)/2} \qquad (1)$$

wherein $R^1$ is a hydrocarbon group having from 1 to 8 carbon atoms, a phenyl group, a hydroxy group, or the formula $-R^2$-Rf ($R^2$ represents a divalent alkylene group having from 2 to 6 carbon atoms, and Rf represents a perfluoroalkyl group having from 1 to 8 carbon atoms) and is optionally selected from the group consisting of a hydroxy group and a functional group in which the formula $-R^2$-Rf is essential, g is the average number, and $1.0 \leq g \leq 1.8$, provided that $R^1$ may be the same or different.

The fluorine-modified silicone resin has an intramolecular silanol group from the viewpoint of reducing the sliding feeling of the emulsion composition on the skin during application, and the proportion of the OH group in the

silanol group is preferably from 0.1 to 5% by mass, more preferably from 0.5 to 5% by mass, based on the resin mass.

The fluorine-modified silicone resin is preferably solid at 25°C. Therefore, from the viewpoint of reducing the sliding feeling of the emulsion composition on the skin during application, the fluorine-modified silicone resin is preferably dissolved in a solvent and used.

As such a fluorine-modified silicone resin, the cosmetic labeling name "trifluoroalkyldimethyltrimethylsiloxysilicate" (INCI name "Trifluoropropyldimethyl/Trimethylsiloxysilicate") is preferred, and a commercial product such as XS66-B8226 (50% by mass decamethylcyclopentasiloxane solution) or XS66-B8636 (50% by mass dimethylpolysiloxane (10 cs) solution) (all manufactured by Momentive Performance Materials Inc.) previously dissolved in a solvent can be used.

As the trimethylsiloxysilicate, a compound having a crosslinked structure having a siloxane structure as the main skeleton and represented by $[(CH_3)_3SiO_{1/2}]_s[SiO_2]_T$ (S is from 1 to 3, and T is from 0.5 to 8) is preferred.

The trimethylsiloxysilicate preferably has a weight average molecular weight of from 1,000 to 10,000, more preferably from 2,000 to 9,000, and further more preferably from 3,000 to 6,000 from the viewpoint of reducing the sliding feeling of the emulsion composition on the skin during application.

The states of the trimethylsiloxysilicate may include any of being liquid, gummy, pasty, solid, and the like at 25°C, but solid trimethylsiloxysilicate is preferred from the viewpoint of reducing the sliding feeling of the emulsion composition on the skin during application.

As the trimethylsiloxysilicate, the cosmetic labeling name "trimethylsiloxysilicate" (INCI name "Trimethylsiloxysilicate") is preferred from the viewpoint of reducing the sliding feeling of the emulsion composition on the skin during application, and a commercial product such as KF-7312T (60% by mass methyl trimethicone solution), KF-7312L (50% dimethylpolysiloxane (2 cs) solution), KF-7312J (50% by mass decamethylcyclopentasiloxane solution), or KF-7312K (50% by mass dimethylpolysiloxane (6 cs) solution) (all manufactured by Shin-Etsu Chemical Co., Ltd.) previously dissolved in a solvent can be used.

Examples of the acrylic silicone resins include vinyl-based polymers having a carbosiloxane dendrimer structure in the side chain, and acrylic-silicone-based graft copolymers.

In the vinyl-based polymer having the carbosiloxane dendrimer structure in the side chain, as the carbosiloxane dendrimer structure, a group of the following formula (2) is preferred from the viewpoint of reducing the sliding feeling of the emulsion composition on the skin during application.

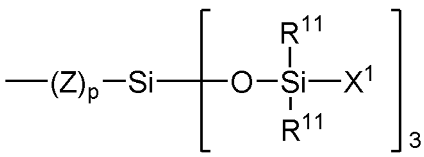 (2)

In the formula, Z is a divalent organic group, p is 0 or 1, and $R^{11}$ is an alkyl group or an aryl group having from 1 to 10 carbon atoms. $R^{11}$ at multiple positions each may be the same or different.

$X^1$ is a silylalkyl group of the following formula when i = 1.

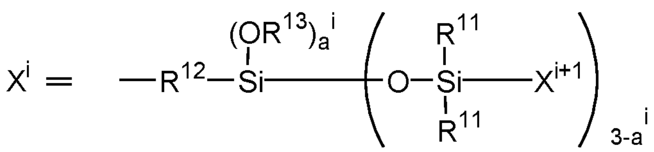

In the formula, $R^{11}$ is the same as above, $R^{12}$ is an alkylene group having from 2 to 10 carbon atoms, and $R^{13}$ is an alkyl group having from 1 to 10 carbon atoms. $R^{11}$ at multiple positions each may be the same or different.

$X^{i+1}$ is a group selected from the group consisting of a hydrogen atom, an alkyl group and an aryl group having from 1 to 10 carbon atoms, and the silylalkyl group. i is an integer of from 1 to 10 indicating the level of the silylalkyl group, and $a^i$ is an integer of from 0 to 3.

In formula (2), Z is a divalent organic group, and examples thereof include alkylene groups, arylene groups, aralkylene groups, ester-containing divalent organic groups, ether-containing divalent organic groups, ketone-containing divalent organic groups, and amide group-containing divalent organic groups. Among these, organic groups of the following formulas are preferred.

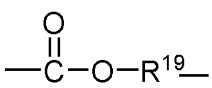 (i)

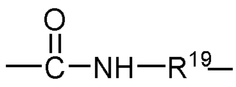

(ii)

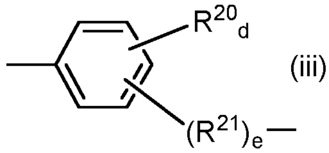

(iii)

In the formulas, $R^{19}$ is an alkylene group having from 1 to 10 carbon atoms, and examples thereof include a methylene group, an ethylene group, a propylene group, and a butylene group. Among these, an ethylene group and a propylene group are preferred. $R^{20}$ is an alkyl group having from 1 to 10 carbon atoms, and examples thereof include a methyl group, an ethyl group, a propyl group, and a butyl group. Among these, a methyl group is preferred. $R^{21}$ is an alkylene group having from 1 to 10 carbon atoms, and examples thereof include alkylene groups such as a methylene group, an ethylene group, a propylene group, and a butylene group. Among these, an ethylene group is preferred. d is an integer of from 0 to 4, and e is 0 or 1.

In formula (2), $R^{11}$ is an alkyl group or an aryl group having from 1 to 10 carbon atoms. Examples of the alkyl group include a methyl group, an ethyl group, a propyl group, a butyl group, a pentyl group, an isopropyl group, an isobutyl group, a cyclopentyl group, and a cyclohexyl group. Examples of the aryl group include a phenyl group and a naphthyl group. Among these, a methyl group and a phenyl group are preferred, and a methyl group is particularly preferred.

$X^1$ is a silylalkyl group of the following formula when i = 1.

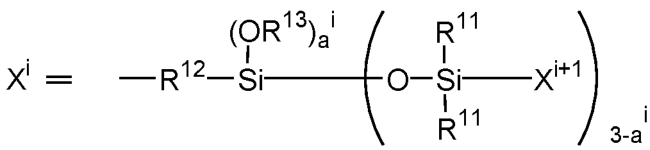

In the formula, $R^{11}$ is the same as above. $R^{12}$ is an alkylene group having from 2 to 10 carbon atoms, and examples thereof include linear alkylene groups such as an ethylene group, a propylene group, a butylene group, and a hexylene group; and branched alkylene groups such as a methylmethylene group, a methylethylene group, a 1-methylpentylene group, and a 1,4-dimethylbutylene group. Among these, an ethylene group, a methylethylene group, a hexylene group, a 1-methylpentylene group, and a 1,4-dimethylbutylene group are preferred. $R^{13}$ is an alkyl group having from 1 to 10 carbon atoms, and examples thereof include a methyl group, an ethyl group, a propyl group, a butyl group, and an isopropyl group. $X^{i+1}$ is a group selected from the group consisting of a hydrogen atom, an alkyl group and an aryl group having from 1 to 10 carbon atoms, and the silylalkyl group. $a^1$ is an integer of from 0 to 3. i is an integer of from 1 to 10, and this indicates the number of levels of the silylalkyl group, that is, the number of repetitions of the silylalkyl group.

As the vinyl-based polymer having the carbosiloxane dendrimer structure in the side chain, preferred is a vinyl-based polymer containing a carbosiloxane dendrimer structure obtained by (co)polymerizing:

(B1) from 0 to 99.9 parts by mass of a vinyl-based monomer other than (B2), and;
(B2)from 100 to 0.1 parts by mass of a carbosiloxane dendrimer having a radically polymerizable organic group of formula (3):

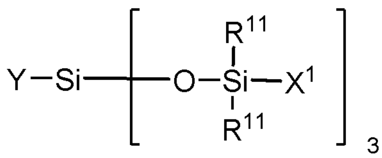

(3)

wherein Y is a radically polymerizable organic group, and $R^{11}$ and $X^1$ are the same as above.

In the above formula, Y is a radically polymerizable organic group, and $R^{11}$ is an alkyl group or an aryl group having from 1 to 10 carbon atoms. Examples of the alkyl group include a methyl group, an ethyl group, a propyl group, a butyl group, a pentyl group, an isopropyl group, an isobutyl group, a cyclopentyl group, and a cyclohexyl group. Examples of the aryl group include a phenyl group and a naphthyl group. Among these, a methyl group and a phenyl

group are preferred, and a methyl group is more preferred.

In this vinyl-based polymer, the (B1) vinyl-based monomer, may have a radically polymerizable vinyl group, and its type and the like are not particularly limited. Examples of such a vinyl-based monomer include lower alkyl (meth)acrylates such as methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, n-hexyl (meth)acrylate, and cyclohexyl (meth)acrylate; higher alkyl (meth)acrylates such as 2-ethylhexyl (meth)acrylate, octyl (meth)acrylate, lauryl (meth)acrylate, and stearyl (meth)acrylate; fatty acid vinyl esters such as vinyl acetate, vinyl propionate, vinyl butyrate, vinyl caproate, vinyl 2-ethylhexanoate, vinyl laurate, and vinyl stearate; aromatic-containing monomers such as styrene, vinyltoluene, benzyl (meth)acrylate, and phenoxyethyl (meth)acrylate; amide group-containing vinyl type monomers such as (meth)acrylamide, N-methylol(meth)acrylamide, N-methoxymethyl(meth)acrylamide, isobutoxymethoxy(meth)acrylamide, N,N-dimethyl(meth)acrylamide, vinylpyrrolidone, and N-vinylacetamide; hydroxy group-containing vinyl type monomers such as hydroxyethyl (meth)acrylate, hydroxypropyl (meth)acrylate, glyceryl (meth)acrylate, and hydroxyethylacrylamide; carboxylic acid-containing vinyl type monomers such as (meth)acrylic acid, itaconic acid, crotonic acid, fumaric acid, and maleic acid, and salts thereof; ether bond-containing vinyl type monomers such as tetrahydrofurfuryl (meth)acrylate, butoxyethyl (meth)acrylate, ethoxydiethylene glycol (meth)acrylate, polyethylene glycol (meth)acrylate, polypropylene glycol mono(meth)acrylate, hydroxybutyl vinyl ether, cetyl vinyl ether, and 2-ethylhexyl vinyl ether; reactive group-containing monomers such as glycidyl (meth)acrylate, (meth)allyl glycidyl ether, methacryloyloxyethyl isocyanate, and (meth)acryloxypropyltrimethoxysilane; macromonomers such as polydimethylsiloxane containing a (meth)acryl group at one end, and polydimethylsiloxane containing a styryl group at one end; butadiene; vinyl chloride; vinylidene chloride; (meth)acrylonitrile; dibutyl fumarate; maleic anhydride; radically polymerizable unsaturated monomers having a sulfonic acid group, such as styrenesulfonic acid and acrylamido-2-methylpropanesulfonic acid, and alkali metal salts, ammonium salts, and organic amine salts thereof; quaternary ammonium salts derived from (meth)acrylic acid, such as 2-hydroxy-3-methacryloxypropyltrimethylammonium chloride; and methacrylates of alcohols having a tertiary amino group, such as diethylaminoethyl methacrylate, vinylpyridine, and quaternary ammonium salts thereof.

A polyfunctional vinyl-based monomer can also be used, and examples thereof include trimethylolpropane tri(meth)acrylate, pentaerythritol tri(meth)acrylate, ethylene glycol di(meth)acrylate, tetraethylene glycol di(meth)acrylate, polyethylene glycol di(meth)acrylate, 1,4-butanediol di(meth)acrylate, 1,6-hexanediol di(meth)acrylate, neopentyl glycol di(meth)acrylate, trimethylolpropane trioxyethyl(meth)acrylate, tris(2-hydroxyethyl)isocyanurate di(meth)acrylate, tris(2-hydroxyethyl)isocyanurate tri(meth)acrylate, and unsaturated group-containing silicone compounds such as styryl group-blocked polydimethylsiloxane.

The component (B2) carbosiloxane dendrimer may be the one having the radically polymerizable organic group of formula (3), and its type and the like are not particularly limited. In formula (3), Y is a radically polymerizable organic group and may be an organic group capable of radical reaction, and specific examples include (meth)acryloxy group-containing organic groups, (meth)acrylamide group-containing organic groups, and styryl group-containing organic groups of the following formulas, and alkenyl groups having from 2 to 10 carbon atoms.

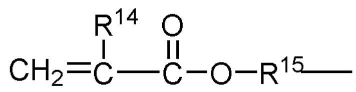

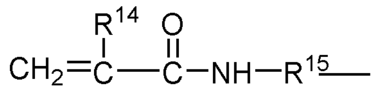

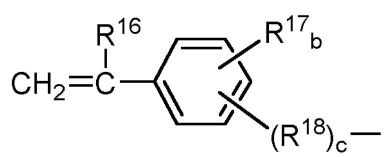

In the formulas, $R^{14}$ and $R^{16}$ are each a hydrogen atom or a methyl group, $R^{15}$ and $R^{18}$ are each an alkylene group having from 1 to 10 carbon atoms, and $R^{17}$ is an alkyl group having from 1 to 10 carbon atoms. b is an integer of from 0 to 4, and c is 0 or 1.

Examples of such a radically polymerizable organic group include a 2-acryloyloxyethyl group, a 3-acryloyloxypropyl group, a 2-methacryloyloxyethyl group, a 3-methacryloyloxypropyl group, a 4-vinylphenyl group, a 3-vinylphenyl group, a 4-(2-propenyl)phenyl group, a 3-(2-propenyl)phenyl group, a 2-(4-vinylphenyl)ethyl group, a 2-(3-vinylphenyl)ethyl group, a vinyl group, an allyl group, a methallyl group, and a 5-hexenyl group.

In formula (3), when i = 1, that is, the number of levels of the silylalkyl group is 1, the (B2) carbosiloxane dendrimer is of formula:

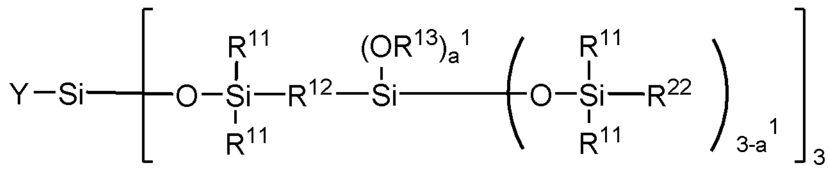

wherein Y, $R^{11}$, $R^{12}$, and $R^{13}$ are the same as above, and $R^{22}$ is a hydrogen atom or the same as the $R^{11}$. $a^1$ is the same as the $a^1$, and the average total number of $a^1$ in one molecule is from 0 to 7.

Examples of such a carbosiloxane dendrimer (B2) containing the radically polymerizable organic group include carbosiloxane dendrimers of the following average composition formulas:

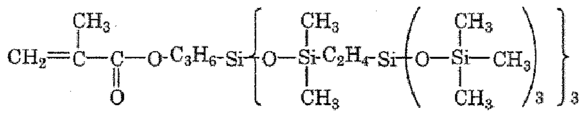

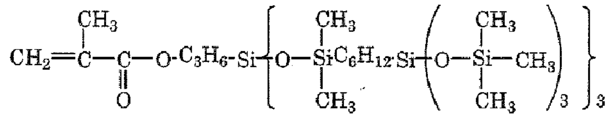

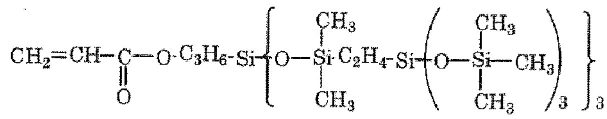

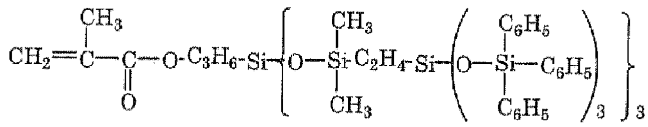

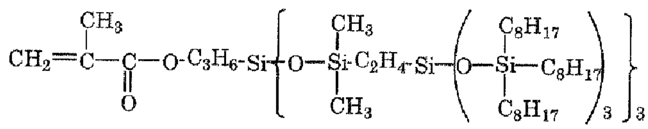

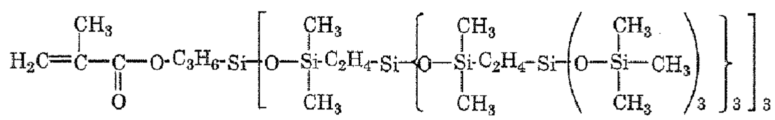

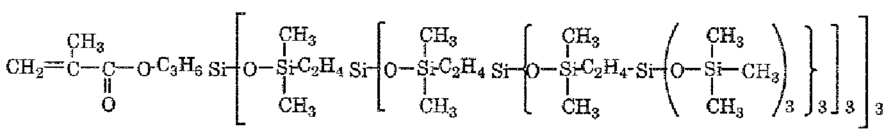

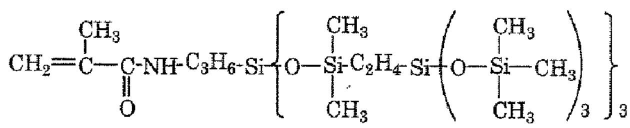

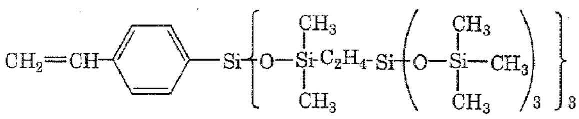

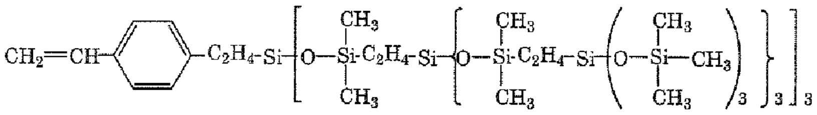

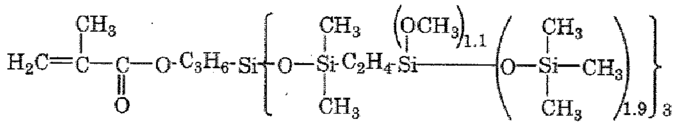

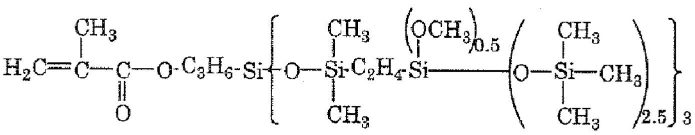

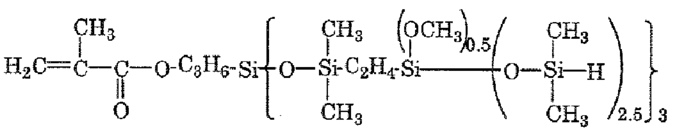

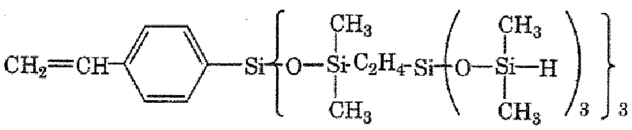

As the component (B2), a carbosiloxane dendrimer of the following average composition formula is preferred from the viewpoint of reducing the sliding feeling of the emulsion composition on the skin during application.

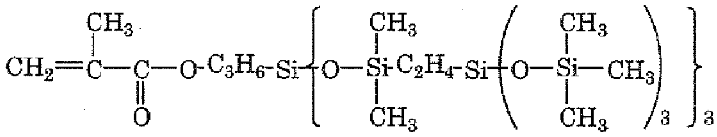

Such a carbosiloxane dendrimer can be produced, for example, in accordance with the production methods described in JP-A-H11-1530, JP-A-2000-63225, and the like.

In the vinyl-based polymer containing the dendrimer structure used in the present invention, the mass ratio of the (B1) component to the (B2) component is preferably (B1):(B2) = from 0:100 to 99.9:0.1, more preferably from 5:95 to 90:10, and further preferably in the range of from 10:90 to 80:20.

The number average molecular weight of the vinyl-based polymer having the carbosiloxane dendrimer structure used in the present invention is preferably from 3,000 to 2,000,000, further more preferably from 5,000 to 800,000, from the viewpoint of reducing the sliding feeling of the emulsion composition on the skin during application.

The states of the vinyl-based polymer may include any of being liquid, gummy, pasty, solid, and the like at 25°C, but a solid one is preferred from the viewpoint of reducing the sliding feeling of the emulsion composition on the skin during application. From the viewpoint of reducing the sliding feeling of the emulsion composition on the skin during application, a solution or a dispersion diluted with a solvent is preferred. Especially, the vinyl-based polymer is preferably used as a dispersion diluted with a liquid oil, and one or more selected from the group consisting of silicone oils and hydrocarbon oils are preferably used, one or more selected from the group consisting of dimethylpolysiloxane, decamethylcyclopentasiloxane, and isododecane are more preferably used, one or more selected from the group consisting

of dimethylpolysiloxane and isododecane are further more preferably used, and dimethylpolysiloxane is even more preferred.

As the vinyl-based polymer having the carbosiloxane dendrimer structure in the side chain, a silicone dendrimer-acrylic copolymer is preferred, the cosmetic labeling name "(acrylates/polytrimethylsiloxymethacrylate) copolymer" (INCI name "Acrylates/Polytrimethylsiloxymethacrylate Copolymer") is preferred, and a commercial product such as FA4001CM (30% by mass decamethylcyclopentasiloxane solution), FA4002ID (40% by mass isododecane solution), or DOWSIL FA 4003 DM Silicone Acrylate (40% by mass dimethylpolysiloxane (2 cs) solution) (all manufactured by Dow Toray Co., Ltd.) previously dissolved in a solvent can be used.

As the acrylic-silicone-based graft copolymer, an acrylic-silicone-based graft copolymer obtained by polymerizing a monomer (B3) containing a dimethylpolysiloxane compound having a radically polymerizable group at one end of the molecular chain of formula (4):

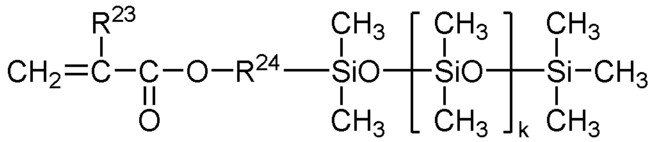

(4)

wherein $R^{23}$ represents a methyl group or a hydrogen atom, $R^{24}$ represents a divalent saturated hydrocarbon group having from 1 to 10 carbon atoms which may be interrupted by one or two ether bonds, and k represents a number of from 3 to 300,

is preferred from the viewpoint of reducing the sliding feeling of the emulsion composition on the skin during application.

In formula (4), the divalent saturated hydrocarbon group having from 1 to 10 carbon atoms which may be interrupted by one or two ether bonds, represented by $R^{24}$, may be either linear or branched, and one having from 1 to 7 carbon atoms is preferred. Specific examples include $-CH_2-$, $-(CH_2)_3-$, $-(CH_2)_6-$, $-(CH_2)_8-$, $-(CH_2)_{10}-$, $-CH_2-CH(CH_3)-CH_2-$, $-CH_2CH_2OCH_2CH_2CH_2-$, $-CH_2CH_2OCH_2CH(CH_3)CH_2-$, and $-CH_2CH_2OCH_2CH_2OCH_2CH_2CH_2-$.

k is a number of from 3 to 300, and is preferably from 5 to 100 from the viewpoint of reducing the sliding feeling of the emulsion composition on the skin during application.

As the dimethylpolysiloxane compound of formula (4), the following can be preferably used from the viewpoint of reducing the sliding feeling of the emulsion composition on the skin during application. Me in the formulas represents a methyl group.

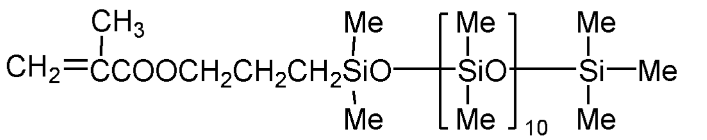

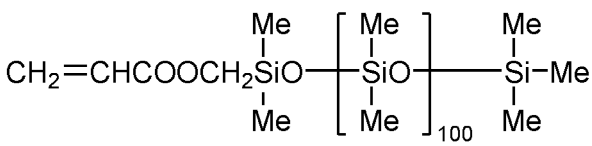

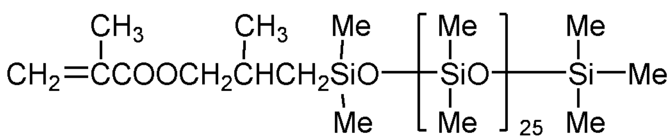

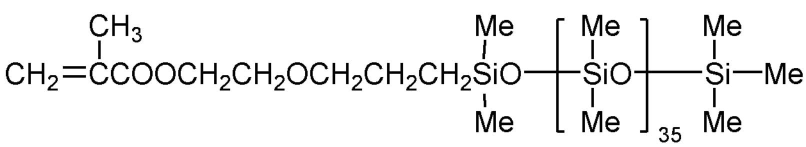

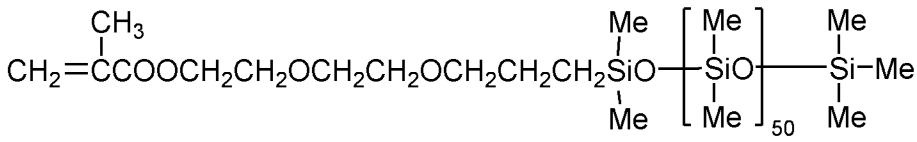

The acrylic-silicone-based graft copolymer is obtained by polymerizing the monomer containing such a dimethylpolysiloxane compound (B3) of formula (4). Examples of the monomer used in addition to the dimethylpolysiloxane compound (B3) include a radically polymerizable monomer (B4) mainly containing an acrylate and/or a methacrylate.

The radically polymerizable monomer mainly containing the acrylate and/or the methacrylate is a compound having one intramolecular radically polymerizable unsaturated bond, and mainly containing the acrylate and/or the methacrylate means that in the radically polymerizable monomer (B4), the total amount of the acrylate and/or the methacrylate accounts for 50% by mass or more of the entire radically polymerizable monomer.

Examples of the acrylate and/or the methacrylate used include alkyl (meth)acrylates such as methyl (meth)acrylate, ethyl (meth)acrylate, butyl (meth)acrylate, and 2-ethylhexyl (meth)acrylate; hydroxyalkyl (meth)acrylates such as 2-hydroxyethyl (meth)acrylate and 2-hydroxypropyl (meth)acrylate; and perfluoroalkyl (meth)acrylates having from 1 to 10 carbon atoms. Especially, methyl methacrylate, butyl methacrylate, and 2-ethylhexyl acrylate are preferred, and a combination of methyl methacrylate, butyl methacrylate, and 2-ethylhexyl acrylate is more preferred.

Examples of the monomer other than the acrylate and/or the methacrylate include styrene, substituted styrene, vinyl acetate, (meth)acrylic acid, maleic anhydride, maleates, fumarates, vinyl chloride, vinylidene chloride, ethylene, propylene, butadiene, acrylonitrile, and fluorinated olefins.

The polymerization of the monomer containing the dimethylpolysiloxane compound (B3) of formula (4) can be performed, for example, in the presence of a radical polymerization initiator by solution polymerization, emulsion polymerization, suspension polymerization, or bulk polymerization, in accordance with an ordinary radical polymerization method.

The obtained acrylic-silicone-based graft copolymer preferably has a weight average molecular weight of from 3,000 to 200,000, more preferably from 5,000 to 100,000.

As the acrylic-silicone-based graft copolymer, one obtained by subjecting the dimethylpolysiloxane compound (B3) of formula (4) and the radically polymerizable monomer (B4) mainly containing the acrylate and/or the methacrylate to radical copolymerization is preferred.

In this case, the polymerization ratio of the dimethylpolysiloxane compound (B3) of formula (4) to the radically polymerizable monomer (B4) mainly containing the acrylate and/or the methacrylate, (B3)/(B4), is preferably from 1/19 to 2/1.

The acrylic-silicone-based graft copolymer is preferably solid at 25°C from the viewpoint of reducing the sliding feeling of the emulsion composition on the skin during application.

From the viewpoint of reducing the sliding feeling of the emulsion composition on the skin during application, the acrylic-silicone-based graft copolymer is preferably dissolved in a solvent and used. Examples of the solvent include silicone oils and hydrocarbon oils. From the viewpoint of reducing the sliding feeling of the emulsion composition on the skin during application, one or more selected from the group consisting of methyl trimethicone, dimethylpolysiloxane (2 cs), decamethylcyclopentasiloxane, and isododecane are preferred, one or more selected from the group consisting of methyl trimethicone, dimethylpolysiloxane (2 cs), and decamethylcyclopentasiloxane are further more preferred, and decamethylcyclopentasiloxane is even more preferred.

As the acrylic-silicone-based graft copolymer, the cosmetic labeling name "(acrylates/dimethicone) copolymer" (INCI name "Acrylates/Dimethicone Copolymer") is preferred, and a commercial product such as KP545 (30% by mass decamethylcyclopentasiloxane solution), KP549 (40% by mass methyl trimethicone solution), or KP550 (40% by mass isododecane solution) (all manufactured by Shin-Etsu Chemical Co., Ltd.) previously dissolved in a solvent can be used.

As the alkylsilsesquioxane resin, for example, the one described in JP-A-H4-312511 can be used. Specific examples can include a silicone resin obtained by reacting from 5 to 100 parts by mass of a silicone compound of $(R^{26}{}_3Si)_{a1}M$, wherein $R^{26}$ represents a substituted or unsubstituted monovalent hydrocarbon group, a1 represents 1 or 2, M represents a hydrogen atom, a hydroxy group, or a hydrolyzable group when a1 is 1, and M represents -O-, -N(X)-, or -S- when a1 is 2; here, X represents a hydrogen atom, a monovalent hydrocarbon group having from 1 to 4 carbon atoms, or $R^{26}{}_3Si$-, with 100 parts by mass of a silanol group-containing organopolysilsesquioxane comprised of an $R^{25}SiO_{1.5}$ unit, wherein $R^{25}$ represents a substituted or unsubstituted monovalent hydrocarbon group, such as a silicone resin containing from 50 to 99 mol% of the $R^{25}SiO_{1.5}$ unit and from 1 to 50 mol% of the $R^{26}{}_3SiO_{0.5}$ unit.

As the alkylsilsesquioxane resin, the cosmetic labeling name "polypropylsilsesquioxanecyclopentasiloxane" (INCI name "POLYPROPYLSILSESQUIOXANECYCLOPENTASILOXANE") is preferred, and as the commercial product, 670FLUID (manufactured by Dow Toray Co., Ltd.), which is polypropylsilsesquioxane, or the like can be used.

The alkylsilsesquioxane resin is preferably used as a mixture with the trimethylsiloxysilicate from the viewpoint

of reducing the sliding feeling of the emulsion composition on the skin during application and improving the feeling of close adhesion to the skin after application.

As the mixture of the alkylsilsesquioxane resin and trimethylsiloxysilicate, a commercial product such as DOWSIL MQ-1640 FLAKE RESIN (mixture of 37% by mass of polypropylsilsesquioxane and 63% by mass of trimethylsiloxysilicate) (manufactured by Dow Toray Co., Ltd.) can be used.

The component (B) film-forming agent preferably contains at least one or more selected from the group consisting of trimethylsiloxysilicate, acrylic silicone resins, and mixtures of alkylsilsesquioxane resins and trimethylsiloxysilicate, more preferably at least one or more selected from the group consisting of trimethylsiloxysilicate, vinyl-based polymers having a carbosiloxane dendrimer structure in the side chain, acrylic-silicone-based graft copolymers, and mixtures of alkylsilsesquioxane resins and trimethylsiloxysilicate, and further more preferably at least one or more selected from the group consisting of vinyl-based polymers having a carbosiloxane dendrimer structure in the side chain, acrylic-silicone-based graft copolymers, and mixtures of alkylsilsesquioxane resins and trimethylsiloxysilicate, and is even more preferably a vinyl-based polymer having a carbosiloxane dendrimer structure in the side chain, from the viewpoint of reducing the sliding feeling of the emulsion composition on the skin during application, improving the feeling of close adhesion to the skin after application and the pore covering ability, and reducing the sticky feeling.

One or more components (B) can be used, and the content is preferably 0.1% by mass or more, more preferably 0.3% by mass or more, further more preferably 0.65% by mass or more, and even more preferably 0.8% by mass or more, and preferably 10% by mass or less, more preferably 7% by mass or less, further more preferably 4% by mass or less, and even more preferably 3.5% by mass or less in the total composition from the viewpoint of reducing the sliding feeling of the emulsion composition on the skin during application, improving the feeling of close adhesion to the skin after application and the pore covering ability, and reducing the sticky feeling. The content of the component (B) is preferably from 0.1 to 10% by mass, more preferably from 0.3 to 7% by mass, even more preferably from 0.65 to 4% by mass, and even more preferably from 0.8 to 3.5% by mass in the total composition.

The component (C) is a nonvolatile oil which is liquid at 25°C.

Being liquid at 25°C indicates that the viscosity at 25°C is 10,000 mPa·s or less. Here, the viscosity is measured with a B type viscometer (TVB-10M, manufactured by TOKI SANGYO CO., LTD.), rotor No. 4, 60 rpm, and 60 s.

Nonvolatile refers to one in which the amount of evaporation at 25°C for 6 h measured by the following method (1) is less than 20%.

Method (1): Filter paper having a diameter of 90 mm is placed in a glass petri dish having a diameter of 120 mm, and 1 g of a sample is placed on the filter paper and stored in a room (25°C) at 65% RH. Then, the residue of the sample after 6 h is measured to calculate the amount of evaporation.

The component (C) nonvolatile oil may be one used in ordinary cosmetics, and examples of the nonvolatile oil include linear or branched hydrocarbon oils such as liquid paraffins, liquid isoparaffins, squalane, and squalene; monoester oils such as ethylhexyl para-methoxycinnamate, octyldodecyl myristate, isotridecyl isononanoate, and isononyl isononanoate; diester oils such as neopentyl glycol dicaprate, propylene glycol dicaprate, and diisostearyl malate; triester oils such as glyceryl tri-2-ethylhexanoate and glyceryl tri(caprylate/caprate); tetraester oils such as dipentaerythrityl tetraisostearate; silicone oils such as dimethylpolysiloxane, caprylyl methicone, methylphenylpolysiloxane, methylhydrogenpolysiloxane, and higher alcohol-modified organopolysiloxanes; and fluorine oils such as fluoropolyethers and perfluoroalkyl ether silicones.

Among these, at least one or more selected from the group consisting of linear or branched hydrocarbon oils, monoester oils, diester oils, triester oils, tetraester oils, and silicone oils are preferably included,

at least one or more selected from the group consisting of linear or branched hydrocarbon oils, monoester oils, diester oils, and silicone oils are more preferably included,
at least one or more selected from the group consisting of branched hydrocarbon oils, monoester oils, diester oils, and silicone oils are further more preferably included, and
at least one or more selected from the group consisting of liquid isoparaffins, ethylhexyl para-methoxycinnamate, methylphenylpolysiloxane, caprylyl methicone, and propylene glycol dicaprate are even more preferably included, from the viewpoint of reducing the sliding feeling of the emulsion composition on the skin during application and improving the feeling of close adhesion to the skin after application and the pore covering ability.

One or more components (C) can be used, and the content is preferably 0.5% by mass or more, more preferably 2% by mass or more, and further more preferably 7% by mass or more, and preferably 35% by mass or less, more preferably 23% by mass or less, and further more preferably 15% by mass or less in the total composition from the viewpoint of reducing the sliding feeling of the emulsion composition on the skin during application and improving the feeling of close adhesion to the skin after application and the pore covering ability. The content of the component (C) is preferably from 0.5 to 35% by mass, more preferably from 2 to 23% by mass, and further more preferably from 7 to 15% by mass in the total composition.

As the component (D) carbonate, a carbonate of a divalent metal is preferred, and examples thereof include calcium carbonate, magnesium carbonate, and barium carbonate. Among these, at least one or more selected from the group consisting of calcium carbonate and magnesium carbonate are preferably included, and calcium carbonate is more preferred, from the viewpoint of reducing the sliding feeling of the emulsion composition on the skin during application and improving the feeling of close adhesion to the skin after application and the pore covering ability. In addition, from the viewpoint of reducing the sticky feeling of the skin after application, at least one or more selected from the group consisting of calcium carbonate and magnesium carbonate are preferably included, and magnesium carbonate is more preferred. Further, from the viewpoint of reducing the sliding feeling of the emulsion composition on the skin during application, improving the feeling of close adhesion to the skin after application and the pore covering ability, and reducing the sticky feeling, calcium carbonate and magnesium carbonate are preferably included in combination.

The component (D) carbonate is used alone. When the carbonate is used as a powder surface treatment agent, or when the carbonate is used as a composite powder raw material, the carbonate is not present alone in the composition, and therefore the effects of the present invention cannot be obtained.

The shape of the component (D) carbonate is not limited and may be any of a spindle shape, a rod shape, a spherical shape, a plate shape, a needle shape, a fiber shape, a petal shape, and the like.

The shape of the component (D) carbonate is preferably a spindle shape, a rod shape, a plate shape, a needle shape, a fiber shape, or a petal shape, more preferably a spindle shape, a rod shape, a plate shape, a needle shape, or a fiber shape, and further more preferably a spindle shape, a rod shape, or a plate shape from the viewpoint of reducing the sliding feeling of the emulsion composition on the skin during application, improving the feeling of close adhesion to the skin after application and the pore covering ability, and reducing the sticky feeling.

The volume average particle diameter of the carbonate is preferably from 0.1 to 50 μm, more preferably from 1 to 30 μm, and even more preferably from 6 to 20 μm from the viewpoint of reducing the sliding feeling of the emulsion composition on the skin during application, improving the feeling of close adhesion to the skin after application and the pore covering ability, and reducing the sticky feeling.

Further, from the viewpoint of reducing the sliding feeling of the emulsion composition on the skin during application, improving the feeling of close adhesion to the skin after application and the pore covering ability, and reducing the sticky feeling, the particle diameter of the long axis with respect to the average thickness (aspect ratio) of the carbonate is preferably 1 or more, more preferably 1.3 or more, and further more preferably 1.6 or more, and preferably 100 or less, more preferably 50 or less, and further more preferably 30 or less. The particle diameter of the long axis with respect to the average thickness (aspect ratio) of the carbonate is preferably from 1 to 100, more preferably from 1.3 to 50, and further more preferably from 1.6 to 30.

Here, the volume average particle diameter is measured by a laser diffraction scattering particle size distribution measuring instrument (manufactured by HORIBA, Ltd., LA-920). In the present invention, the volume average particle diameter is the volume-based average particle diameter and is the 50% median diameter.

The aspect ratio is calculated by the ratio of the volume average particle diameter to the average thickness of the particles and defined by aspect ratio = (volume average particle diameter/average thickness).

The average thickness of the particles is obtained by number-averaging the thickness of from 10 to 50 mother particles measured by observation with a scanning electron microscope or a transmission electron microscope.

As the component (D) carbonate, a commercial product can be used. Examples of calcium carbonate include light calcium carbonate (manufactured by Ohmi Chemical Industry Co., Ltd., volume average particle diameter 7.5 μm, spindle shape, aspect ratio 1.9) and SCS-M5 (manufactured by Sakai Chemical Industry Co., Ltd., volume average particle diameter 5 μm, spherical shape, aspect ratio 1). Examples of magnesium carbonate include heavy magnesium carbonate (manufactured by Kyowa Chemical Industry Co., Ltd., volume average particle diameter 16 μm, plate shape, aspect ratio 26).

One or more components (D) can be used, and the content is preferably 0.1% by mass or more, more preferably 0.5% by mass or more, and further more preferably 1.2% by mass or more, and preferably 10% by mass or less, more preferably 8% by mass or less, and further more preferably 5% by mass or less in the total composition from the viewpoint of reducing the sliding feeling of the emulsion composition on the skin during application, improving the feeling of close adhesion to the skin after application and the pore covering ability, and reducing the sticky feeling. The content of the component (D) is preferably from 0.1 to 10% by mass, more preferably from 0.5 to 8% by mass, and further more preferably from 1.2 to 5% by mass in the total composition.

In the present invention, a mass ratio of the component (B) to the component (D), (B)/(D), is preferably 0.02 or more, more preferably 0.05 or more, further more preferably 0.1 or more, and even more preferably 0.2 or more, and preferably 50 or less, more preferably 20 or less, further more preferably 10 or less, and even more preferably 2.5 or less from the viewpoint of reducing the sliding feeling of the emulsion composition on the skin during application, improving the feeling of close adhesion to the skin after application and the pore covering ability, and reducing the sticky feeling. The mass ratio of the component (B) to the component (D), (B)/(D), is preferably from 0.02 to 50, more preferably from 0.05 to 20, further more preferably from 0.1 to 10, and even more preferably from 0.2 to 2.5.

In the present invention, a mass ratio of the component (C) to the component (D), (C)/(D), is preferably 0.1 or more, more preferably 0.4 or more, further more preferably 1 or more, and preferably 100 or less, more preferably 50 or less, and further more preferably 12 or less from the viewpoint of reducing the sliding feeling of the emulsion composition on the skin during application, improving the feeling of close adhesion to the skin after application and the pore covering ability, and reducing the sticky feeling. The mass ratio of the component (C) to the component (D), (C)/(D), is preferably from 0.1 to 100, more preferably from 0.4 to 50, and further more preferably from 1 to 12.

In the present invention, the content of the component (E) water, is preferably 20% by mass or more, more preferably 30% by mass or more, further more preferably 40% by mass or more, and even more preferably 45% by mass or more, and preferably 80% by mass or less, more preferably 70% by mass or less, further more preferably 60% by mass or less, and even more preferably 55% by mass or less in the total composition. The content of the component (E) is preferably from 20 to 80% by mass, more preferably from 30 to 70% by mass, further more preferably from 40 to 60% by mass, and even more preferably from 45 to 55% by mass in the total composition.

The emulsion composition of the present invention can further contain a coloring pigment.

The coloring pigment is one used in ordinary cosmetics, and examples of the coloring pigment include inorganic pigments such as metal oxides such as titanium oxide, zinc oxide, cerium oxide, aluminum oxide, yellow iron oxide, black iron oxide, red oxide, Prussian blue, ultramarine blue, chromium oxide, and chromium hydroxide, metal complexes such as manganese violet and cobalt titanate, and further carbon black; synthetic organic pigments such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 401, Blue No. 1, and Blue No. 404; and natural organic dyes such as β-carotin, caramel, and paprika dye, and include composites of these coloring pigments, and composite pigments in which these coloring pigments are combined with pearl pigments. Examples of the pearl pigments to be treated include natural or synthetic inorganic powders such as mica, phlogopite, talc, silica, sericite, glass, kaolin, bismuth oxychloride, cerium oxide, calcium sulfate, barium sulfate, magnesium sulfate, and plate-shaped alumina powders. Specific examples of the composite pigments include titanium oxide-coated mica, iron oxide-coated mica, iron oxide-coated mica titanium, iron oxide-coated synthetic phlogopite, chromium oxide-coated mica titanium, titanium oxide-coated glass powders, iron oxide-coated glass powders, titanium oxide-contained glass powders, and iron oxide-contained glass powders.

Among these, metal oxides are preferred, at least one or more selected from the group consisting of titanium oxide, zinc oxide, yellow iron oxide, black iron oxide, and red oxide are more preferably included, and at least one or more selected from the group consisting of titanium oxide, yellow iron oxide, black iron oxide, and red oxide are further more preferably included.

These coloring pigments can be used as they are, or they may be also subjected to hydrophobization treatment and used. Examples of the hydrophobization treatment include surface treatment such as fluorine compound treatment, silicone treatment, alkyl treatment, alkylsilane treatment, metallic soap treatment, watersoluble polymer treatment, amino acid treatment, acylated amino acid treatment, lecithin treatment, organotitanate treatment, polyol treatment, acrylic resin treatment, methacrylic resin treatment, and urethane resin treatment.

As the hydrophobization treatment, hydrophobization treatment by at least one or more selected from the group consisting of silicone treatment, amino acid treatment, and acylated amino acid treatment is preferred, and hydrophobization treatment by at least one or more selected from the group consisting of silicone treatment and acylated amino acid treatment is more preferred, from the viewpoint of reducing the sliding feeling of the emulsion composition on the skin during application, improving the feeling of close adhesion to the skin after application and the pore covering ability, and reducing the sticky feeling. From the viewpoint of reducing the sliding feeling of the emulsion composition on the skin during application, improving the feeling of close adhesion to the skin after application and the pore covering ability, and reducing the sticky feeling, silicone treatment and acylated amino acid treatment are preferably used in combination.

The hydrophobization treatment of the coloring pigment can be performed by an ordinary method.

One or more coloring pigments can be used, and the content is preferably from 1 to 30% by mass, more preferably from 3 to 25% by mass, further more preferably from 5 to 20% by mass, and even more preferably from 7 to 15% by mass in the total composition from the viewpoint of reducing the sliding feeling of the emulsion composition on the skin during application, improving the feeling of close adhesion to the skin after application and the pore covering ability, and reducing the sticky feeling.

The emulsion composition of the present invention can further contain an oil agent which is solid or semisolid at 25°C, and can reduce the sliding feeling of the emulsion composition on the skin during application and improve the feeling of close adhesion to the skin after application and the pore covering ability.

Being solid or semisolid at 25°C means that the viscosity at 25°C is greater than 10,000 mPa·s.

Here, the viscosity is measured with a B type viscometer (model TVB-10, manufactured by TOKI SANGYO CO., LTD.), rotor No. 4, 12 rpm, and 60 s (rotor No. 4, 3 rpm, and 60 s for 50,000 mmPa.s or more).

The oil agent which is solid at 25°C is not limited as long as it is usually used in cosmetics. An ester wax, a hydrocarbon wax, or the like can be used. Examples of the oil agent which is solid at 25°C containing an ester wax

include animal waxes and vegetable waxes, and examples of the oil agent which is solid at 25°C containing a hydrocarbon wax include mineral waxes and synthetic waxes. More specifically, examples of the oil agent which is solid at 25°C containing an ester wax include shea butter, rice bran wax, carnauba wax, candelilla wax, and beeswax, which are vegetable waxes, and examples of the oil agent which is solid at 25°C containing a hydrocarbon wax include ceresin, paraffin waxes, microcrystalline waxes, polyethylene waxes, and polyolefin waxes.

Examples of the oil agent which is semisolid at 25°C include cholesterol derivatives such as cholesteryl isostearate, cholesteryl hydroxystearate, cholesteryl macadamiate, and di(cholesteryl-behenyl-octyldodecyl) N-lauroyl-L-glutamate; phytosterol derivatives such as di(phytosteryl-behenyl-2-octyldodecyl) N-lauroyl-L-glutamate, di(phytosteryl-2-octyldodecyl) N-lauroyl-L-glutamate, phytosteryl isostearate, phytosteryl oleate, and (phytosteryl/isostearyl/cetyl/stearyl/behenyl) dimer dilinoleate; dipentaerythritol fatty acid esters such as dipentaerythritol hexaoxystearate and dipentaerythritol rosinate; triglycerides such as tri(caprylic-capric-myristic-stearic acid) glyceride, glyceryl trilanolate, and glyceryl tribehenate; partially hydrogenated triglycerides such as hydrogenated oils, lanolin, soft lanolin acid, lanosterol derivatives, Vaseline, and dimer dilinoleic acid hydrogenated castor oil.

The oil agent which is solid or semisolid at 25°C preferably contains at least one or more selected from the group consisting of vegetable waxes, cholesterol derivatives, phytosterol derivatives, triglycerides, lanolin, Vaseline, and dimer dilinoleic acid hydrogenated castor oil, more preferably at least one or more selected from the group consisting of vegetable waxes, cholesterol derivatives, phytosterol derivatives, triglycerides, lanolin, and Vaseline, is further more preferably a vegetable wax, a phytosterol derivative, or a triglyceride, and even more preferably contains a triglyceride, from the viewpoint of reducing the sliding feeling of the emulsion composition on the skin during application, improving the feeling of close adhesion to the skin after application and the pore covering ability, and reducing the sticky feeling.

One or more oil agents which are solid or semisolid at 25°C can be used, and the content is preferably from 0.05 to 8% by mass, more preferably from 0.06 to 5% by mass, and further more preferably from 0.07 to 3% by mass in the total composition from the viewpoint of reducing the sliding feeling of the emulsion composition on the skin during application, improving the feeling of close adhesion to the skin after application and the pore covering ability, and reducing the sticky feeling.

The emulsion composition of the present invention can further contain a volatile oil and can reduce the sliding feeling of the emulsion composition on the skin during application.

Here, the volatile oil refers to one in which the amount of evaporation at 25°C for 6 h measured by the following method (1) is 20% or more.

Method (1): Filter paper having a diameter of 90 mm is placed in a glass petri dish having a diameter of 120 mm, and 1 g of a sample is placed on the filter paper and stored in a room (25°C) at 65% RH. Then, the residue of the sample after 6 h is measured to calculate the amount of evaporation.

The volatile oil may be any used in ordinary cosmetics, and examples of the volatile oil include silicone oils, hydrocarbon oils, and ether oils.

Examples of the silicone oils include chain dimethylpolysiloxane and cyclic dimethylpolysiloxane. The chain dimethylpolysiloxane may be either linear or branched. Examples of the linear dimethylpolysiloxane include dimethylpolysiloxane (1.5 cs) and dimethylpolysiloxane (2 cs). Examples of the branched dimethylpolysiloxane include methyl trimethicone, tris(trimethylsilyl)methylsilane, and tetrakis(trimethylsilyl)silane. Examples of the cyclic dimethylpolysiloxane include octamethylcyclotetrasiloxane, decamethylcyclopentasiloxane, and dodecamethylcyclohexasiloxane.

Examples of the hydrocarbon oils include isododecane, isotridecane, isohexadecane, light isoparaffins, and light liquid isoparaffins, and examples of the ether oils include ethyl perfluorobutyl ether.

Among these, at least one or more selected from the group consisting of silicone oils and hydrocarbon oils are preferably included, at least one or more selected from the group consisting of dimethylpolysiloxane (2 cs), decamethylcyclopentasiloxane, isododecane, and light liquid isoparaffins are more preferably included, and at least one or more selected from the group consisting of dimethylpolysiloxane (2 cs), decamethylcyclopentasiloxane, and light liquid isoparaffins are even more preferably included, from the viewpoint of reducing the sliding feeling of the emulsion composition on the skin during application.

One or more volatile oils can be used, and the content is preferably from 1 to 30% by mass, more preferably from 3 to 20% by mass, and further more preferably from 5 to 15% by mass in the total composition from the viewpoint of reducing the sliding feeling of the emulsion composition on the skin during application.

In the emulsion composition of the present invention, from the viewpoint of environmental consideration and an excellent use impression, the content of an organic resin powder is preferably 1% by mass or less, more preferably 0.1% by mass or less, in the total composition, and the emulsion composition of the present invention further more preferably contains substantially no organic resin powder.

The content of a spherical organic resin powder is preferably 1% by mass or less, more preferably 0.1% by mass or less, in the total composition, and the emulsion composition of the present invention further more preferably contains substantially no spherical organic resin powder.

Examples of the organic resin powder include crosslinked or non-crosslinked organic resin powders such as

powders of one or more polymers or copolymers selected from the group consisting of polyamide resins, nylon resins, polyester resins, polyethylene resins, polytetrafluoroethylene resins, polypropylene resins, polystyrene resins, benzoguanamine resins, polymethylbenzoguanamine resins, polyurethane resins, vinyl resins, fluororesins, acrylic resins, and melamine resins; butyl acrylate-vinyl acetate copolymers, styrene-acrylic acid copolymers, silicone resins, and divinylbenzene-styrene copolymers.

The emulsion composition of the present invention can contain, in addition to the components, components usually used in cosmetics, for example, an extender pigment such as talc, a powder other than the above, a polymer compound, an antioxidant, a perfume, a preservative, a pH adjusting agent, a blood circulation promoter, a cold sense agent, an antiperspirant, a disinfectant, a skin activator, a moisturizer, and a refrigerant.

The emulsion composition of the present invention can be produced in accordance with an ordinary method, and any of a water-in-oil emulsion composition, an oil-in-water emulsion composition, and the like can be formed. A water-in-oil emulsion composition is preferred.

The emulsion composition of the present invention can be in a liquid, emulsion, pasty, creamy, gel, or solid form or the like and is preferably pasty, creamy, or solid.

The emulsion composition of the present invention can be applied as water-in-oil emulsion cosmetics and can be applied as makeup cosmetics such as cosmetic bases, foundations, concealers, blushers, eye shadows, mascaras, eyeliners, eyebrow pencils, overcoat agents, and lipsticks; ultraviolet protecting cosmetics such as sunscreen emulsions and sunscreen creams; and the like. The emulsion composition of the present invention is preferred as makeup cosmetics.

Regarding the embodiment, the present invention further discloses the following compositions.

<1> An emulsion composition comprising the following components (A), (B), (C), (D), and (E):

(A) a lipophilic nonionic surfactant;
(B) a film-forming agent;
(C) a nonvolatile oil which is liquid at 25°C;
(D) a carbonate; and
(E) water.

<2> The emulsion composition according to <1>, wherein the component (A) preferably has an HLB of from 1 to 8, more preferably from 2 to 7, and further more preferably from 3 to 6.

<3> The emulsion composition according to <1> or <2>, wherein the component (A) preferably comprises at least one or more selected from the group consisting of sorbitan fatty acid esters and silicone-based surfactants, more preferably at least one or more selected from the group consisting of sorbitan fatty acid esters and polyether-modified silicones, further more preferably at least one or more selected from the group consisting of sorbitan monoisostearate, sorbitan monooleate, sorbitan sesquistearate, and polyether-modified silicones, and even more preferably at least one or more selected from the group consisting of sorbitan monoisostearate and polyoxyethylene-methylpolysiloxane copolymers.

<4> The emulsion composition according to any one of <1> to <3>, wherein in the component (A), a sorbitan fatty acid ester and a silicone-based surfactant are preferably used in combination.

<5> The emulsion composition according to any one of <1> to <4>, wherein a content of the component (A) is preferably 1% by mass or more, more preferably 1.5% by mass or more, and further more preferably 2% by mass or more, and preferably 8% by mass or less, more preferably 6.5% by mass or less, and further more preferably 5.5% by mass or less in a total composition.

<6> The emulsion composition according to any one of <1> to <5>, wherein the component (B) is preferably one or more selected from the group consisting of fluorine-modified silicone resins, trimethylsiloxysilicate, acrylic silicone resins, and alkylsilsesquioxane resins, more preferably comprises at least one or more selected from the group consisting of trimethylsiloxysilicate, acrylic silicone resins, and mixtures of alkylsilsesquioxane resins and trimethylsiloxysilicate, further more preferably at least one or more selected from the group consisting of trimethylsiloxysilicate, vinyl-based polymers having a carbosiloxane dendrimer structure in the side chain, acrylic-silicone-based graft copolymers, and mixtures of alkylsilsesquioxane resins and trimethylsiloxysilicate, and even more preferably at least one or more selected from the group consisting of vinyl-based polymers having a carbosiloxane dendrimer structure in the side chain, acrylic-silicone-based graft copolymers, and mixtures of alkylsilsesquioxane resins and trimethylsiloxysilicate, and is even more preferably a vinyl-based polymer having a carbosiloxane dendrimer structure in the side chain.

<7> The emulsion composition according to any one of <1> to <6>, wherein a content of the component (B) is preferably 0.1% by mass or more, more preferably 0.3% by mass or more, further more preferably 0.65% by mass or more, and even more preferably 0.8% by mass or more, and preferably 10% by mass or less, more preferably 7% by mass or less, further more preferably 4% by mass or less, and even more preferably 3.5% by mass or less

in the total composition.

<8> The emulsion composition according to any one of <1> to <7>, wherein the component (C) preferably comprises at least one or more selected from the group consisting of linear or branched hydrocarbon oils, monoester oils, diester oils, triester oils, tetraester oils, and silicone oils, more preferably at least one or more selected from the group consisting of linear or branched hydrocarbon oils, monoester oils, diester oils, and silicone oils, further more preferably at least one or more selected from the group consisting of branched hydrocarbon oils, monoester oils, diester oils, and silicone oils, and even more preferably at least one or more selected from the group consisting of liquid isoparaffins, ethylhexyl para-methoxycinnamate, methylphenylpolysiloxane, caprylyl methicone, and propylene glycol dicaprate.

<9> The emulsion composition according to any one of <1> to <8>, wherein a content of the component (C) is preferably 0.5% by mass or more, more preferably 2% by mass or more, and further more preferably 7% by mass or more, and preferably 35% by mass or less, more preferably 23% by mass or less, and further more preferably 15% by mass or less in the total composition.

<10> The emulsion composition according to any one of <1> to <9>, wherein the component (D) preferably comprises at least one or more selected from the group consisting of calcium carbonate and magnesium carbonate.

<11> The emulsion composition according to any one of <1> to <10>, wherein in the component (D), calcium carbonate and magnesium carbonate are preferably used in combination.

<12> The emulsion composition according to any one of <1> to <11>, wherein a content of the component (D) is preferably 0.1% by mass or more, more preferably 0.5% by mass or more, and further more preferably 1.2% by mass or more, and preferably 10% by mass or less, more preferably 8% by mass or less, and further more preferably 5% by mass or less in the total composition.

<13> The emulsion composition according to any one of <1> to <12>, wherein a mass ratio of the component (B) to the component (D), (B)/(D), is preferably 0.02 or more, more preferably 0.05 or more, further more preferably 0.1 or more, and even more preferably 0.2 or more, and preferably 50 or less, more preferably 20 or less, further more preferably 10 or less, and even more preferably 2.5 or less.

<14> The emulsion composition according to any one of <1> to <13>, wherein a mass ratio of the component (C) to the component (D), (C)/(D), is preferably 0.1 or more, more preferably 0.4 or more, further more preferably 1 or more, and preferably 100 or less, more preferably 50 or less, and further more preferably 12 or less.

<15> The emulsion composition according to any one of <1> to <14>, wherein a content of the component (E) water is preferably 20% by mass or more, more preferably 30% by mass or more, further more preferably 40% by mass or more, and even more preferably 45% by mass or more, and preferably 80% by mass or less, more preferably 70% by mass or less, further more preferably 60% by mass or less, and even more preferably 55% by mass or less in the total composition.

<16> The emulsion composition according to any one of <1> to <15>, preferably further comprising a coloring pigment, wherein the coloring pigment is preferably a metal oxide and more preferably comprises at least one or more selected from the group consisting of titanium oxide, zinc oxide, yellow iron oxide, black iron oxide, and red oxide, further more preferably at least one or more selected from the group consisting of titanium oxide, yellow iron oxide, black iron oxide, and red oxide.

<17> The emulsion composition according to <16>, wherein the coloring pigment is preferably subjected to hydrophobization treatment, more preferably hydrophobization treatment by at least one or more selected from the group consisting of silicone treatment, amino acid treatment, and acylated amino acid treatment, and further more preferably hydrophobization treatment by at least one or more selected from the group consisting of silicone treatment and acylated amino acid treatment.

<18> The emulsion composition according to <16> or <17>, wherein a content of the coloring pigment is preferably from 1 to 30% by mass, more preferably from 3 to 25% by mass, further more preferably from 5 to 20% by mass, and even more preferably from 7 to 15% by mass in the total composition.

<19> The emulsion composition according to any one of <1> to <18>, preferably further comprising an oil agent which is solid or semisolid at 25°C, wherein the oil agent which is solid or semisolid at 25°C preferably comprises at least one or more selected from the group consisting of vegetable waxes, cholesterol derivatives, phytosterol derivatives, triglycerides, lanolin, Vaseline, and dimer dilinoleic acid hydrogenated castor oil, more preferably at least one or more selected from the group consisting of vegetable waxes, cholesterol derivatives, phytosterol derivatives, triglycerides, lanolin, and Vaseline, is further more preferably a vegetable wax, a phytosterol derivative, or a triglyceride, and even more preferably comprises a triglyceride.

<20> The emulsion composition according to <19>, wherein a content of the oil agent which is solid or semisolid at 25°C is preferably from 0.05 to 8% by mass, more preferably from 0.06 to 5% by mass, and further more preferably from 0.07 to 3% by mass in the total composition.

<21> The emulsion composition according to any one of <1> to <20>, preferably further comprising a volatile oil, wherein the volatile oil preferably comprises at least one or more selected from the group consisting of silicone oils

and hydrocarbon oils, more preferably at least one or more selected from the group consisting of dimethylpolysiloxane (2 cs), decamethylcyclopentasiloxane, isododecane, and light liquid isoparaffins, and even more preferably at least one or more selected from the group consisting of dimethylpolysiloxane (2 cs), decamethylcyclopentasiloxane, and light liquid isoparaffins.

<22> The emulsion composition according to <21>, wherein a content of the volatile oil is preferably from 1 to 30% by mass, more preferably from 3 to 20% by mass, and further more preferably from 5 to 15% by mass in the total composition.

<23> The water-in-oil emulsion composition according to any one of <1> to <22>, wherein a content of an organic resin powder is preferably 1% by mass or less, more preferably 0.1% by mass or less, in the total composition, the water-in-oil emulsion composition further more preferably comprising substantially no organic resin powder.

<24> The water-in-oil emulsion composition according to any one of <1> to <23>, wherein a content of a spherical organic resin powder is preferably 1% by mass or less, more preferably 0.1% by mass or less, in the total composition, the water-in-oil emulsion composition further more preferably comprising substantially no spherical organic resin powder.

<25> The emulsion composition according to any one of <1> to <24>, being preferably a water-in-oil emulsion composition, more preferably a water-in-oil emulsion cosmetic.

<26> An emulsion composition comprising the following components (A), (B), (C), (D), and (E):

(A) from 1 to 8% by mass of at least one or more lipophilic nonionic surfactants selected from the group consisting of sorbitan fatty acid esters and silicone-based surfactants;
(B) from 0.1 to 10% by mass of a film-forming agent;
(C) from 0.5 to 35% by mass of a nonvolatile oil which is liquid at 25°C;
(D) from 0.1 to 10% by mass of at least one or more carbonates selected from the group consisting of calcium carbonate and magnesium carbonate; and
(E) water.

<27> An emulsion composition comprising the following components (A), (B), (C), (D), and (E):

(A) from 1 to 8% by mass of at least one or more lipophilic nonionic surfactants selected from the group consisting of sorbitan fatty acid esters and silicone-based surfactants;
(B) from 0.1 to 10% by mass of a film-forming agent;
(C) from 0.5 to 35% by mass of a nonvolatile oil which is liquid at 25°C;
(D) from 0.1 to 10% by mass of at least one or more carbonates selected from the group consisting of calcium carbonate and magnesium carbonate; and
(E) water, and

from 1 to 30% by mass of a coloring pigment.

<28> An emulsion composition comprising the following components (A), (B), (C), (D), and (E):

(A) from 2 to 5.5% by mass of at least one or more lipophilic nonionic surfactants selected from the group consisting of sorbitan fatty acid esters and silicone-based surfactants;
(B) from 0.8 to 3.5% by mass of a film-forming agent;
(C) from 0.5 to 35% by mass of a nonvolatile oil which is liquid at 25°C;
(D) from 1.2 to 5% by mass of at least one or more carbonates selected from the group consisting of calcium carbonate and magnesium carbonate; and
(E) water.

<29> An emulsion composition comprising the following components (A), (B), (C), (D), and (E):

(A) from 1 to 8% by mass of at least one or more lipophilic nonionic surfactants selected from the group consisting of sorbitan fatty acid esters and silicone-based surfactants;
(B) from 0.1 to 10% by mass of at least one or more film-forming agents selected from the group consisting of trimethylsiloxysilicate, acrylic silicone resins, and mixtures of alkylsilsesquioxane resins and trimethylsiloxysilicate;
(C) from 0.5 to 35% by mass of a nonvolatile oil which is liquid at 25°C;
(D) from 0.1 to 10% by mass of at least one or more carbonates selected from the group consisting of calcium carbonate and magnesium carbonate; and
(E) water.

<30> An emulsion composition comprising the following components (A), (B), (C), (D), and (E):

(A) from 2 to 5.5% by mass of at least one or more lipophilic nonionic surfactants selected from the group consisting of sorbitan fatty acid esters and silicone-based surfactants;
(B) from 0.8 to 3.5% by mass of at least one or more film-forming agents selected from the group consisting of trimethylsiloxysilicate, acrylic silicone resins, and mixtures of alkylsilsesquioxane resins and trimethylsiloxysilicate;
(C) from 0.5 to 35% by mass of a nonvolatile oil which is liquid at 25°C;
(D) from 1.2 to 5% by mass of at least one or more carbonates selected from the group consisting of calcium carbonate and magnesium carbonate; and
(E) water.

<31> An emulsion composition comprising the following components (A), (B), (C), (D), and (E):

(A) from 2 to 5.5% by mass of at least one or more lipophilic nonionic surfactants selected from the group consisting of sorbitan fatty acid esters and silicone-based surfactants;
(B) from 0.8 to 3.5% by mass of at least one or more film-forming agents selected from the group consisting of trimethylsiloxysilicate, acrylic silicone resins, and mixtures of alkylsilsesquioxane resins and trimethylsiloxysilicate;
(C) from 0.5 to 35% by mass of a nonvolatile oil which is liquid at 25°C;
(D) from 1.2 to 5% by mass of at least one or more carbonates selected from the group consisting of calcium carbonate and magnesium carbonate; and
(E) water, wherein
a mass ratio of the component (B) to the component (D), (B)/(D), is from 0.1 to 10.

<32> Use of the emulsion composition according to any one of <1> to <31> as an emulsion cosmetic.
<33> Use of the emulsion composition according to any one of <1> to <31> as a water-in-oil emulsion composition.
<34> Use of the emulsion composition according to any one of <1> to <31> as a water-in-oil emulsion cosmetic.
<35> Use of the emulsion composition according to any one of <1> to <31> as a makeup cosmetic.
<36> Use for makeup of the emulsion composition according to any one of <1> to <31>, which is applied to a skin, preferably a face.
<37> Use of the emulsion composition according to any one of <1> to <31> for the production of the emulsion composition.

Examples

Examples 1 to 12 and Comparative Example 1

Water-in-oil emulsion cosmetics having the compositions shown in Table 1 and Table 2 were produced, and the absence of a sliding feeling during application, the feeling of close adhesion to the skin after application, the pore covering ability, and the absence of stickiness of the skin after application were evaluated. The results are shown together in Table 1 and Table 2.

(Production Method)

Components (A), (B), and (C) and other oily components were heated with stirring at 80°C to prepare an oil phase mixture, and a previously pulverized and mixed powder phase was added to the oil phase mixture and mixed using a homomixer. Subsequently, a water phase heated to 80°C was added, mixed, and then cooled to room temperature to obtain a water-in-oil emulsion cosmetic.

(Evaluation Methods)

(1) Absence of Sliding Feeling during Application:

When three expert evaluators took 0.3 g of each water-in-oil emulsion cosmetic on a finger and applied it to the face, they evaluated the absence of a sliding feeling during application by the following criteria. The result is shown by the total points of the three expert evaluators.
The sliding feeling indicates that when the water-in-oil emulsion cosmetic is taken on a fingertip or the like and

applied to the face, there is a feeling that the water-in-oil emulsion cosmetic spreads for a long period of time on the skin as if a liquid oil agent were spread, and there is no actual feeling that the water-in-oil emulsion cosmetic spreads while adhering to the skin.

5; The evaluator clearly feels no sliding feeling.
4; The evaluator hardly feels a sliding feeling.
3; The evaluator does not feel a sliding feeling very much.
2; The evaluator somewhat feels a sliding feeling.
1; The evaluator clearly feels a sliding feeling.

(2) Feeling of Close Adhesion to Skin after Application:

Three expert evaluators took 0.3 g of each water-in-oil emulsion cosmetic on a finger, applied it to the face, spread it, and then evaluated the feeling of close adhesion to the skin after application by the following criteria. The result is shown by the total points of the three expert evaluators.

The feeling of close adhesion indicates that the water-in-oil emulsion composition attached to the skin after application follows the movement of the skin but does not come off the surface of the skin even if the skin of the face is moved by a change in expression, or the like; and the water-in-oil emulsion composition does not gather partially in the form of large wrinkles, small wrinkles, and the like; and the water-in-oil emulsion composition adheres firmly.

5; The evaluator clearly feels a feeling of close adhesion.
4; The evaluator considerably feels a feeling of close adhesion.
3; The evaluator feels a feeling of close adhesion.
2; The evaluator does not feel a feeling of close adhesion much.
1; The evaluator clearly feels no feeling of close adhesion.

(3) Pore Covering Ability:

Three expert evaluators took 0.3 g of each water-in-oil emulsion cosmetic on a finger, applied it to the face, spread it, and then evaluated the pore covering ability by the following criteria. The result is shown by the total points of the three expert evaluators.

5; The evaluator feels that the pores are clearly hidden.
4; The evaluator feels that the pores are hidden.
3; The evaluator feels that the pores are substantially hidden.
2; The evaluator feels that the pores are not hidden so much.
1; The evaluator feels that the pores are clearly not hidden.

(4) Absence of Stickiness of Skin after Application:

Three expert evaluators took 0.3 g of each water-in-oil emulsion cosmetic on a finger, applied it to the face, then touched the skin after application, and evaluated the absence of stickiness by the following criteria. The result is shown by the total points of the three expert evaluators.

5; The evaluator clearly feels no stickiness.
4; The evaluator hardly feels stickiness.
3; The evaluator does not feel stickiness very much.
2; The evaluator somewhat feels stickiness.
1; The evaluator clearly feels stickiness.

[Table 1]

| Component (% by mass) | | | | Example 1 | Example 2 | Example 3 | Example 4 | Example 5 | Example 6 | Example 7 | Comparative Example 1 |
|---|---|---|---|---|---|---|---|---|---|---|---|
| Oil phase | (A) | Sorbitan monoisostearate | *1 | 0.5 | 0.5 | 0.5 | 0.5 | 0.5 | 0.5 | 0.5 | 0.5 |
| | (A) | Polyoxyethylene-methylpolysiloxane copolymer | *2 | 4 | 4 | 4 | 4 | 2 | 4 | 4 | 4 |
| | (B) | (Acrylates/polytrimethylsiloxymethacrylate) copolymer-dimethicone mixture (40% by mass dimethylpolysiloxane (2 cs) solution) | *3 | 7 | 7 | 7 | | | | 7 | 7 |
| | (B) | (Acrylates/dimethicone) copolymer-decamethylcyclopentasiloxane mixed liquid (30% by mass decamethylcyclopentasiloxane solution) | *4 | | | | 2 | 2 | | | |
| | (B) | Polypropylsilsesquioxane-trimethylsiloxysilicate mixture (mixture of 37% by mass of polypropylsilsesquioxane and 63% by mass of trimethylsiloxysilicate) | *5 | | | | | | 0.5 | | |
| | (C) | Liquid isoparaffin | | 0.5 | 0.5 | 0.5 | 0.5 | 2 | 0.5 | 0.5 | 0.5 |
| | (C) | 2-Ethylhexyl para-methoxycinnamate | *6 | | | | | 4 | | | |
| | (C) | Methylphenylpolysiloxane | *7 | 4 | 4 | 4 | 4 | 3 | 4 | 4 | 4 |
| | (C) | Caprylyl methicone | | 5 | 5 | 5 | 5 | 5 | 5 | 5 | 5 |
| | (C) | Propylene glycol dicaprate | | 1.5 | 1.5 | 1.5 | 1.5 | 1 | 1.5 | 1.5 | 1.5 |
| | | Dimethylpolysiloxane (2cs) | *8 | 5.5 | 5.5 | 5.5 | 10.5 | 1 | 12 | 5.5 | 5.5 |
| | | Decamethylcyclopentasiloxane | | | | | | 5 | | | |
| | | Light liquid isoparaffin | *9 | 1.5 | 1.5 | 1.5 | 1.5 | 2 | 1.5 | 1.5 | 1.5 |
| | | Glyceryl tribehenate | *10 | 0.1 | 0.1 | 0.1 | 0.1 | 2 | 0.1 | 0.1 | 0.1 |
| Water phase | | Glycerin | | 1 | 1 | 1 | 1 | | 1 | 1 | 1 |
| | | Dipropylene glycol | | 4 | 4 | 4 | 4 | 4 | 4 | 4 | 4 |
| | | Diglycerin | | | | | | 1 | | | |
| | | Sodium chloride | | 0.5 | 0.5 | 0.5 | 0.5 | 0.5 | 0.5 | 0.5 | 0.5 |
| | | Phenoxyethanol | | 0.5 | 0.5 | 0.5 | 0.5 | 0.5 | 0.5 | 0.5 | 0.5 |
| | (E) | Purified water | | 47.9 | 47.9 | 47.9 | 47.9 | 48.6 | 47.9 | 47.9 | 47.9 |
| Powder phase | | Dimethicone-disodium stearoyl glutamate-composite-treated titanium oxide | *11 | 10 | 10 | 10 | 10 | 7 | 10 | 10 | 10 |
| | | Hydrogen dimethicone-treated titanium oxide | *12 | | | | | 0.5 | | | |
| | | Dimethicone-disodium stearoyl glutamate-composite-treated red oxide | *13 | 0.3 | 0.3 | 0.3 | 0.3 | 0.2 | 0.3 | 0.3 | 0.3 |
| | | Dimethicone-disodium stearoyl glutamate-composite-treated yellow iron oxide | *14 | 1.3 | 1.3 | 1.3 | 1.3 | 0.9 | 1.3 | 1.3 | 1.3 |
| | | Dimethicone-disodium stearoyl glutamate-composite-treated black iron oxide | *15 | 0.15 | 0.15 | 0.15 | 0.15 | 0.12 | 0.15 | 0.15 | 0.15 |
| | | Dimethicone-disodium stearoyl glutamate-composite-treated talc | *16 | 2.25 | 2.25 | 2.25 | 2.25 | 2.18 | 2.25 | 2.25 | 2.25 |
| | | Dimethicone-treated silica | *17 | 0.5 | 0.5 | 0.5 | 0.5 | 0.5 | 0.5 | 0.5 | 2.5 |
| | (D) | Calcium carbonate | *18 | 0.5 | 2 | | 2 | 3 | 2 | | |
| | (D) | Calcium carbonate | *19 | | | | | | | 2 | |
| | (D) | Magnesium carbonate | *20 | 1.5 | | 2 | | 1.5 | | | |
| | | Total | | 100.00 | 100.00 | 100.00 | 100.00 | 100.00 | 100.00 | 100.00 | 100.00 |
| | | (A) Total amount | | 4.50 | 4.50 | 4.50 | 4.50 | 2.50 | 4.50 | 4.50 | 4.50 |
| | | (B) Total amount | | 2.80 | 2.80 | 2.80 | 0.60 | 0.60 | 0.50 | 2.80 | 2.80 |
| | | (C) Total amount | | 11.00 | 11.00 | 11.00 | 11.00 | 15.00 | 11.00 | 11.00 | 11.00 |
| | | (D) Total amount | | 2.00 | 2.00 | 2.00 | 2.00 | 4.50 | 2.00 | 2.00 | 0.00 |
| | | (B)/(D) | | 1.40 | 1.40 | 1.40 | 0.30 | 0.13 | 0.25 | 1.40 | - |
| | | (C)/(D) | | 5.50 | 5.50 | 5.50 | 5.50 | 3.33 | 5.50 | 5.50 | - |
| | | Absence of sliding feeling during application | | 15 | 15 | 13 | 13 | 13 | 13 | 12 | 3 |
| | | Feeling of close adhesion to skin after application | | 15 | 15 | 12 | 15 | 13 | 13 | 12 | 10 |
| | | Pore covering ability | | 14 | 14 | 13 | 13 | 12 | 13 | 13 | 5 |
| | | Absence of stickiness of skin after application | | 14 | 13 | 15 | 13 | 12 | 13 | 14 | 6 |

[Table 2]

| Component (% by mass) | | | | Example 8 | Example 9 | Example 10 | Example 11 | Example 12 |
|---|---|---|---|---|---|---|---|---|
| Oil phase | (A) | Sorbitan monoisostearate | *1 | 0.5 | 0.5 | 0.5 | 0.5 | 0.5 |
| | (A) | Polyoxyethylene-methylpolysiloxane copolymer | *2 | 4 | 4 | 4 | 4 | 4 |
| | (B) | (Acrylates/polytrimethylsiloxymethacrylate) copolymer-dimethicone mixture (40% by mass dimethylpolysiloxane (2 cs) solution) | *3 | 1.75 | 15 | 7 | 7 | 7 |
| | (C) | Liquid isoparaffin | | 0.5 | 0.5 | 0.5 | 0.5 | 0.5 |
| | (C) | Methylphenylpolysiloxane | *7 | 4 | 4 | 4 | 4 | 4 |
| | (C) | Caprylyl methicone | | 5 | 5 | 5 | 5 | 5 |
| | (C) | Propylene glycol dicaprate | | 1.5 | 1.5 | 1.5 | 1.5 | 1.5 |
| | | Dimethylpolysiloxane (2cs) | *8 | 10 | 0 | 5.5 | 5.5 | 5.6 |
| | | Light liquid isoparaffin | *9 | 1.5 | 1.5 | 1.5 | 1.5 | 1.5 |
| | | Glyceryl tribehenate | *10 | 0.1 | 0.1 | 0.1 | 0.1 | - |
| Water phase | | Glycerin | | 1 | 1 | 1 | 1 | 1 |
| | | Dipropylene glycol | | 4 | 4 | 4 | 4 | 4 |
| | | Sodium chloride | | 0.5 | 0.5 | 0.5 | 0.5 | 0.5 |
| | | Phenoxyethanol | | 0.5 | 0.5 | 0.5 | 0.5 | 0.5 |
| | (E) | Purified water | | 48.65 | 45.4 | 49.1 | 45.9 | 45.9 |
| Powder phase | | Dimethicone-disodium stearoyl glutamate-composite-treated titanium oxide | *11 | 10 | 10 | 10 | 10 | 10 |
| | | Dimethicone-disodium stearoyl glutamate-composite-treated red oxide | *13 | 0.3 | 0.3 | 0.3 | 0.3 | 0.3 |
| | | Dimethicone-disodium stearoyl glutamate-composite-treated yellow iron oxide | *14 | 1.3 | 1.3 | 1.3 | 1.3 | 1.3 |
| | | Dimethicone-disodium stearoyl glutamate-composite-treated black iron oxide | *15 | 0.15 | 0.15 | 0.15 | 0.15 | 0.15 |
| | | Dimethicone-disodium stearoyl glutamate-composite-treated talc | *16 | 2.25 | 2.25 | 2.25 | 2.25 | 2.25 |
| | | Dimethicone-treated silica | *17 | 0.5 | 0.5 | 0.5 | 0.5 | 0.5 |
| | (D) | Calcium carbonate | *18 | 0.5 | 0.5 | 0.2 | 1 | 1 |
| | (D) | Magnesium carbonate | *20 | 1.5 | 1.5 | 0.6 | 3 | 3 |
| | | Total | | 100.00 | 100.00 | 100.00 | 100.00 | 100.00 |
| | | (A) Total amount | | 4.50 | 4.50 | 4.50 | 4.50 | 4.50 |
| | | (B) Total amount | | 0.70 | 6.00 | 2.80 | 2.80 | 2.80 |
| | | (C) Total amount | | 11.00 | 11.00 | 11.00 | 11.00 | 11.00 |
| | | (D) Total amount | | 2.00 | 2.00 | 0.80 | 4.00 | 4.00 |
| | | (B)/(D) | | 0.35 | 3.00 | 3.50 | 0.70 | 0.70 |
| | | (C)/(D) | | 5.50 | 5.50 | 13.75 | 2.75 | 2.75 |
| | | Absence of sliding feeling during application | | 15 | 12 | 13 | 14 | 14 |
| | | Feeling of close adhesion to skin after application | | 13 | 15 | 10 | 14 | 12 |
| | | Pore covering ability | | 14 | 13 | 11 | 14 | 14 |
| | | Absence of stickiness of skin after application | | 14 | 15 | 13 | 15 | 14 |

*1: sorbitan monoisostearate: manufactured by Croda, Span 120,

*2: polyoxyethylene-methylpolysiloxane copolymer: manufactured by Shin-Etsu Chemical Co., Ltd., KF-6017,

*3: (acrylates/polytrimethylsiloxymethacrylate) copolymer dimethicone mixture (40% by mass dimethylpolysiloxane (2 cs) solution): manufactured by Dow Toray Co., Ltd., DOWSIL FA 4003 DM Silicone Acrylate,

*4: (acrylates/dimethicone) copolymer-decamethylcyclopentasiloxane mixed liquid (30% by mass decamethylcyclopentasiloxane solution): manufactured by Shin-Etsu Chemical Co., Ltd.: KP-545,

*5: polypropylsilsesquioxane-trimethylsiloxysilicate mixture (mixture of 37% by mass of polypropylsilsesquioxane and 63% by mass of trimethylsiloxysilicate): manufactured by Dow Toray Co., Ltd.: DOWSIL MQ-1640 FLAKE RESIN,

*6: 2-ethylhexyl para-methoxycinnamate: manufactured by BASF, Uvinul MC80,

*7: methylphenylpolysiloxane: manufactured by Dow Toray Co., Ltd., DOWSIL FZ-209,

*8: dimethylpolysiloxane (2 cs): manufactured by Shin-Etsu Chemical Co., Ltd., KF-96L-2 cs,

*9: light liquid isoparaffin: manufactured by NOF CORPORATION, PARLEAM 4,

*10: glyceryl tribehenate: manufactured by Croda, Syncrowax HR-C,

*11: dimethicone-disodium stearoyl glutamate-composite-treated titanium oxide: manufactured by Miyoshi Kasei, Inc., SA/NAI-TR-10 MIBRID COLOR POWDER,

*12: hydrogen dimethicone-treated titanium oxide: manufactured by DAITO KASEI KOGYO CO., LTD., SI01-2 TIO2 MT-500SA,

*13: dimethicone-disodium stearoyl glutamate-composite-treated red oxide: manufactured by Miyoshi Kasei, Inc., SA/NAI-R-10 MIBRID COLOR POWDER,

*14: dimethicone-disodium stearoyl glutamate-composite-treated yellow iron oxide: manufactured by Miyoshi Kasei, Inc., SA/NAI-Y-10 MIBRID COLOR POWDER,

*15: dimethicone-disodium stearoyl glutamate-composite-treated black iron oxide: manufactured by Miyoshi Kasei, Inc., SA/NAI-B-10 MIBRID COLOR POWDER,

*16: dimethicone-disodium stearoyl glutamate-composite-treated talc: manufactured by Miyoshi Kasei, Inc., SA/NAI-TA-46R MIBRID COLOR POWDER,

*17: dimethicone-treated silica: manufactured by Miyoshi Kasei, Inc., A-SB-300 (7%),

*18: calcium carbonate: manufactured by Ohmi Chemical Industry Co., Ltd., light calcium carbonate (volume average particle diameter 7.5 μm, spindle shape, aspect ratio 1.9)

*19: spherical calcium carbonate: manufactured by Sakai Chemical Industry Co., Ltd., SCS-M5, (volume average particle diameter 5 μm, spherical shape, aspect ratio 1)

*20: magnesium carbonate: manufactured by Kyowa Chemical Industry Co., Ltd., heavy magnesium carbonate (volume average particle diameter 16 μm, plate shape, aspect ratio 26)

Formulation Examples 1 to 4

Water-in-oil emulsion cosmetics having the compositions shown in Table 3 were produced in the same manner as Examples 1 to 12.

The obtained water-in-oil emulsion cosmetics all have no sliding feeling during application, a feeling of close adhesion to the skin after application, excellent pore covering ability, and no stickiness of the skin after application.

[Table 3]

| Phase | | Component (% by mass) | | Formulation Example 1 | Formulation Example 2 | Formulation Example 3 | Formulation Example 4 |
|---|---|---|---|---|---|---|---|
| Oil phase | (A) | Sorbitan monoisostearate | *1 | 0.5 | 0.5 | 0.5 | 0.5 |
| | (A) | Polyoxyethylene-methylpolysiloxane copolymer | *2 | 4 | 4 | 4 | 4 |
| | (B) | (Acrylates/polytrimethylsiloxymethacrylate) copolymer-dimethicone mixture (40% by mass dimethylpolysiloxane (2 cs) solution) | *3 | 7 | 7 | 7 | 7 |
| | (C) | Liquid isoparaffin | | 0.05 | 0.2 | 0.7 | 1.5 |
| | (C) | Methylphenylpolysiloxane | *7 | 0.36 | 1.5 | 6 | 11 |
| | (C) | Caprylyl methicone | | 0.45 | 1.8 | 7.2 | 13.5 |
| | (C) | Propylene glycol dicaprate | | 0.14 | 0.5 | 2.1 | 4 |
| | | Dimethylpolysiloxane (2cs) | *8 | 5.5 | 5.5 | 5.5 | 5.5 |
| | | Light liquid isoparaffin | *9 | 1.5 | 1.5 | 1.5 | 1.5 |
| | | Glyceryl tribehenate | *10 | 0.1 | 0.1 | 0.1 | 0.1 |
| Water phase | | Glycerin | | 1 | 1 | 1 | 1 |
| | | Dipropylene glycol | | 4 | 4 | 4 | 4 |
| | | Sodium chloride | | 0.5 | 0.5 | 0.5 | 0.5 |
| | | Phenoxyethanol | | 0.5 | 0.5 | 0.5 | 0.5 |
| | (E) | Purified water | | 57.9 | 54.9 | 42.9 | 28.9 |
| Powder phase | | Dimethicone-disodium stearoyl glutamate-composite-treated titanium oxide | *11 | 10 | 10 | 10 | 10 |
| | | Dimethicone-disodium stearoyl glutamate-composite-treated red oxide | *13 | 0.3 | 0.3 | 0.3 | 0.3 |
| | | Dimethicone-disodium stearoyl glutamate-composite-treated yellow iron oxide | *14 | 1.3 | 1.3 | 1.3 | 1.3 |
| | | Dimethicone-disodium stearoyl glutamate-composite-treated black iron oxide | *15 | 0.15 | 0.15 | 0.15 | 0.15 |
| | | Dimethicone-disodium stearoyl glutamate-composite-treated talc | *16 | 2.25 | 2.25 | 2.25 | 2.25 |
| | | Dimethicone-treated silica | *17 | 0.5 | 0.5 | 0.5 | 0.5 |
| | (D) | Calcium carbonate | *18 | 0.5 | 0.5 | 0.5 | 0.5 |
| | (D) | Magnesium carbonate | *20 | 1.5 | 1.5 | 1.5 | 1.5 |
| | | Total | | 100.00 | 100.00 | 100.00 | 100.00 |
| | | (A) Total amount | | 4.50 | 4.50 | 4.50 | 4.50 |
| | | (B) Total amount | | 2.80 | 2.80 | 2.80 | 2.80 |
| | | (C) Total amount | | 1.00 | 4.00 | 16.00 | 30.00 |
| | | (D) Total amount | | 2.00 | 2.00 | 2.00 | 2.00 |
| | | (B)/(D) | | 1.40 | 1.40 | 1.40 | 1.40 |
| | | (C)/(D) | | 0.50 | 2.00 | 8.00 | 15.00 |

## Claims

1. An emulsion composition comprising the following components (A), (B), (C), (D), and (E):

(A) a lipophilic nonionic surfactant;

(B) a film-forming agent;
(C) a nonvolatile oil which is liquid at 25°C;
(D) a carbonate; and
(E) water.

2. The emulsion composition according to claim 1, wherein a content of the component (A) is from 1 to 8% by mass.

3. The emulsion composition according to claim 1 or 2, wherein a content of the component (B) is from 0.1 to 10% by mass.

4. The emulsion composition according to any one of claims 1 to 3, wherein a content of the component (C) is from 0.5 to 35% by mass.

5. The emulsion composition according to any one of claims 1 to 4, wherein a content of the component (D) is from 0.1 to 10% by mass.

6. The emulsion composition according to any one of claims 1 to 5, wherein the component (A) comprises at least one or more selected from the group consisting of sorbitan fatty acid esters and silicone-based surfactants.

7. The emulsion composition according to any one of claims 1 to 6, wherein the component (D) comprises at least one or more selected from the group consisting of calcium carbonate and magnesium carbonate.

8. The emulsion composition according to any one of claims 1 to 7, wherein the component (B) is at least one or more selected from the group consisting of trimethylsiloxysilicate, acrylic silicone resins, and mixtures of alkylsilsesquioxane resins and trimethylsiloxysilicate.

9. The emulsion composition according to any one of claims 1 to 8, wherein the component (B) is a vinyl-based polymer having a carbosiloxane dendrimer structure in its side chain.

10. The emulsion composition according to any one of claims 1 to 9, wherein a mass ratio of the component (B) to the component (D), (B)/(D), is from 0.02 to 50.

11. The emulsion composition according to any one of claims 1 to 10, wherein a mass ratio of the component (C) to the component (D), (C)/(D), is from 0.1 to 100.

12. The emulsion composition according to any one of claims 1 to 11, wherein a content of a coloring pigment is from 1 to 30% by mass.

13. The emulsion composition according to any one of claims 1 to 12, being a water-in-oil emulsion cosmetic.

14. Use of the emulsion composition according to any one of claims 1 to 13 as a makeup cosmetic.

15. Use for makeup of the emulsion composition according to any one of claims 1 to 13, which is applied to a skin, preferably a face.

**INTERNATIONAL SEARCH REPORT**

International application No.
**PCT/JP2021/037568**

**A. CLASSIFICATION OF SUBJECT MATTER**

***A61K 8/37***(2006.01)i; ***A61K 8/06***(2006.01)i; ***A61K 8/19***(2006.01)i; ***A61K 8/31***(2006.01)i; ***A61K 8/49***(2006.01)i; ***A61K 8/891***(2006.01)i; ***A61K 8/894***(2006.01)i; ***A61Q 1/02***(2006.01)i
FI: A61K8/37; A61K8/06; A61K8/19; A61K8/31; A61K8/49; A61K8/891; A61K8/894; A61Q1/02

According to International Patent Classification (IPC) or to both national classification and IPC

**B. FIELDS SEARCHED**

Minimum documentation searched (classification system followed by classification symbols)

A61K8/37; A61K8/06; A61K8/19; A61K8/31; A61K8/49; A61K8/891; A61K8/894; A61Q1/02

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2011-1332 A (SHIN-ETSU CHEMICAL CO LTD) 06 January 2011 (2011-01-06) claim 1, paragraphs [0017]-[0018], [0097], [0115], [0121], [0162]-[0163] | 1-15 |
| Y | JP 2017-66092 A (KAO CORP) 06 April 2017 (2017-04-06) claims, paragraphs [0007], [0029], table 1 | 1-15 |
| Y | JP 2013-103885 A (KOSE CORP) 30 May 2013 (2013-05-30) paragraphs [0026]-[0028] | 1-15 |
| Y | JP 2010-180205 A (KOSE CORP) 19 August 2010 (2010-08-19) paragraphs [0066]-[0067] | 1-15 |
| A | JP 2017-178887 A (KOSE CORP) 05 October 2017 (2017-10-05) entire text | 1-15 |
| A | JP 2012-246446 A (DOW CORNING TORAY CO LTD) 13 December 2012 (2012-12-13) entire text | 1-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 December 2021** | **21 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | Telephone No. |

Form PCT/ISA/210 (second sheet) (January 2015)

**INTERNATIONAL SEARCH REPORT**
**Information on patent family members**

International application No.
**PCT/JP2021/037568**

| Patent document cited in search report | Publication date (day/month/year) | Patent family member(s) | Publication date (day/month/year) |
|---|---|---|---|
| JP 2011-1332 A | 06 January 2011 | EP 2266532 A2<br>claim 1, paragraphs [0016]-[0018], [0069], [0130], [0198]-[0200] | |
| JP 2017-66092 A | 06 April 2017 | (Family: none) | |
| JP 2013-103885 A | 30 May 2013 | (Family: none) | |
| JP 2010-180205 A | 19 August 2010 | (Family: none) | |
| JP 2017-178887 A | 05 October 2017 | (Family: none) | |
| JP 2012-246446 A | 13 December 2012 | US 2014/0187649 A1<br>entire text<br>WO 2012/165233 A1<br>EP 2716685 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014172872 A
- JP 2011236182 A
- JP 2020506953 A
- JP H111530 A
- JP 2000063225 A
- JP H4312511 A